(19) Europäisches Patentamt / European Patent Office / Office européen des brevets

(11) EP 4 617 354 A1

(12) **EUROPÄISCHE PATENTANMELDUNG**

(43) Veröffentlichungstag:
**17.09.2025 Patentblatt 2025/38**

(21) Anmeldenummer: **25161326.1**

(22) Anmeldetag: **03.03.2025**

(51) Internationale Patentklassifikation (IPC):
***C12M 1/34*** *(2006.01)* ***C12M 1/36*** *(2006.01)*
***C12Q 1/04*** *(2006.01)*

(52) Gemeinsame Patentklassifikation (CPC):
**C12M 41/48; C12M 41/32; C12M 41/34; C12M 41/36; C12Q 1/04**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **07.03.2024 DE 102024106637**

(71) Anmelder: **Ingenieurbüro Kraus & Kraus**
**13465 Berlin (DE)**

(72) Erfinder: **KRAUS, Mirjam**
**87779 Trunkelsberg (DE)**

(74) Vertreter: **Wehlan, Martin**
**Patentanwälte Wehlan & Wehlan**
**Möllendorffstraße 49**
**10367 Berlin (DE)**

(54) **VERFAHREN UND MITTEL ZUR BESTIMMUNG DER WACHSTUMSRATE IN EINER KULTIVIERUNGSVORRICHTUNG FÜR MIKROORGANISMEN**

(57) Die Erfindung betrifft ein Verfahren und ein Mittel zur Bestimmung der spezifischen Wachstumsrate ($\mu$) in einer Kultivierungsvorrichtung für Mikroorganismen. Aus der Messung eines Energie-Äquivalents im Gas-Zustrom der Kultivierungsvorrichtung und der Messung eines Energie-Äquivalents im Abgas der Kultivierungsvorrichtung wird aus deren Differenz die in der Kultivierungsvorrichtung erfolgte Energieaufnahme (EUR) ermittelt. Alternativ kann die Energieaufnahme (EUR) auch durch den Verbrauch einer Energiequelle (z.B. Glukose) innerhalb der Kulturvorrichtung mit entsprechendem Sensor ermittelt werden. Die Auswertung der ermittelten Energieaufnahme erfolgt nach einem mathematischen Modell, welches auf dem realen Verhalten der Auslaufgeschwindigkeit eines Behälters mit Zulauf und konstanter Auslauföffnung am Boden des Behälters, vorzugsweise ein Regenfass, in Abhängigkeit vom Druck der Flüssigkeitssäule basiert. Gegenstand der Erfindung ist auch ein universelles mathematisches Modell zur Beschreibung von Wachstumsprozessen in Kultivierungsvorrichtungen für Mikroorganismen, insbesondere zur Ermittlung des Zusammenhangs zwischen Zell-Wachstum und Zell-Erhaltung, das gemessene Werte für Energieäquivalente so auswertet, als ob die Wachstumsprozesse einem realen Verhalten der Auslaufgeschwindigkeit eines Behälters mit Zulauf und konstanter Auslauföffnung am Boden des Behälters in Abhängigkeit vom Druck der Flüssigkeitssäule entsprechen.

Figur 1

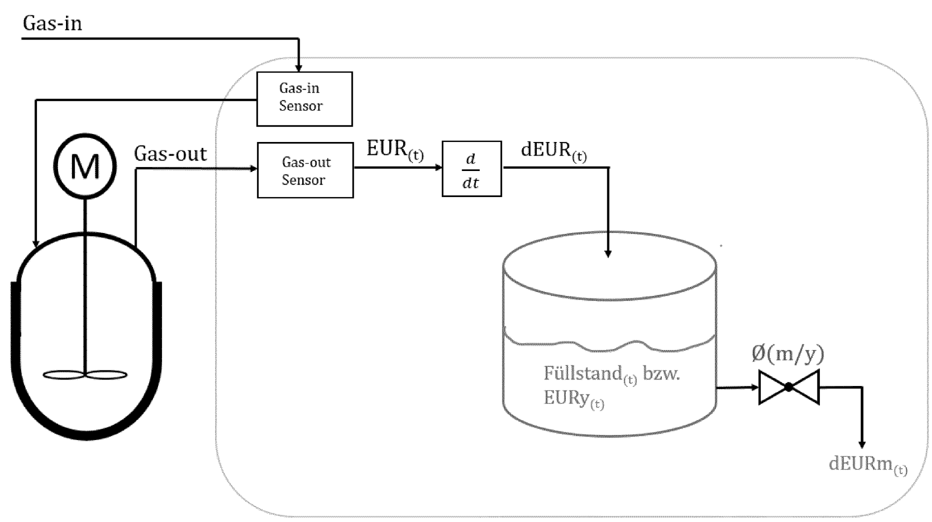


EP 4 617 354 A1

## Beschreibung

Die Erfindung betrifft ein Verfahren und ein Mittel zur Bestimmung der Wachstumsrate ($\mu$) in einer Kultivierungsvorrichtung für Mikroorganismen. Aus der Messung eines Energie-Äquivalents im Gas-Zustrom der Kultivierungsvorrichtung und der Messung eines Energie-Äquivalents im Abgas der Kultivierungsvorrichtung wird aus deren Differenz die in der Kultivierungsvorrichtung erfolgte Energieaufnahme (EUR) ermittelt. Alternativ kann die Energieaufnahme (EUR) auch durch die Konzentrationsänderung eines Energie-Äquivalents (z.B. die Glukose-Konzentration) innerhalb der Mikroorganismen-Kultur mit entsprechendem Sensor ermittelt werden. Die Auswertung der ermittelten Energieaufnahme erfolgt nach einem mathematischen Modell, welches auf dem realen Verhalten der Auslaufgeschwindigkeit eines Behälters mit Zulauf und konstanter Auslauföffnung am Boden des Behälters, vorzugsweise ein Regenfass, in Abhängigkeit vom Druck der Flüssigkeitssäule basiert. Ein mögliches Anwendungsgebiet dieses Verfahrens und Mittels ist die Zellwachstumsbestimmung in Bioreaktoren.

## Stand der Technik

1) Wichtigkeit der Zellwachstumsbestimmung

Die spezifische Wachstumsrate der Biomasse ($\mu$), ist nach der FDA (U.S. Food and Drug Administration) eine Schlüsselvariable für die Bioprozessoptimierung in Folge ihres direkten Einflusses auf die Produkt-Qualität und Quantität. Stand der Technik zur Beschreibung mikrobiellen Wachstums in Bioprozessen basiert u.a. auf dem Luedeking-Piret-Modell (LPG) aus dem Jahr 1959 [Literaturstellen 1-3]. Dieses Modell konnte sich wegen der übersichtlichen Struktur, allgemeinen Anwendbarkeit und guten Nachbildung von Bioprozessen als hilfreiches Werkzeug in der Biotechnologie etablieren.

2) Die Luedeking-Piret-Gleichung (LPG)

Die LPG berechnet die spezifische Wachstumsrate ($\mu$) über die nachfolgenden Gleichungen:

a)

$$X_{(t)} = X_{(0)} * e^{\mu_{(t)} * \Delta t} \qquad \text{Gleichung 1 (Wachstumsmodell der Population)}$$

b)

$$\mu_{(t)} = \frac{EUR_{(t)}/X_{(t)}}{y} - \frac{m}{y} \qquad \text{Gleichung 2}$$

Hierbei werden die folgenden Modellparameter benötigt: die Energieaufnahme proportionale Messgröße (EUR) als Energieäquivalent, die Biomasse (X) sowie die zwei Konstanten Biomasse-Erhaltungskoeffizient (m) und wahrer Biomasse-Energie-Ausbeutekoeffizient (y).

Die Messgröße (EUR), kann in die Anteile EURy und EURm aufgeteilt werden: c)

$$EUR = EURy + EURm \qquad \text{Gleichung 3 (Summengleichung)}$$

Dabei entspricht EURy der momentanen Energie, die für die Zellteilung benötigt wird und EURm der momentanen Energie, die für die Erhaltung der bestehenden und der neugebildeten Zellen benötigt wird. Nach der LPG definieren sich diese Anteile wie folgt:

d)

$$EURy = \mu * X * y \qquad \text{Gleichung 4}$$

e)

$$EURm = X * m \qquad \text{Gleichung 5}$$

3) Problematik der Luedeking-Piret-Gleichung (LPG)

Die LPG zeigt jedoch in der praktischen Anwendung zur Echtzeit-Bestimmung der spezifischen Wachstumsrate ($\mu$) Schwierigkeiten. Dies beeinflusst (A) die Einfachheit der Bedienung für den Endverbraucher und (B) die Genauigkeit der Zielgrößen-Berechnung.

Der Grund für die Schwierigkeiten (A) und (B), ist, dass die LPG auf einem allgemeinen Wachstumsmodell der Population basiert. Das heißt, für die exakte Berechnung von $\mu$ über die LPG ist es notwendig, zu bestimmten Zeitpunkten des Bioprozesses, die exakte Biomasse (X) im Bioreaktor zu kennen. Genauer bedeutet dies, die Biomasse wird als Startwerteingabe ($X_0$) und für die Bestimmung der mikroorganismus- und bioprozessspezifischen Modellparameter (y und m) der LPG benötigt.

4) Die Bestimmung der Biomasse (X)

Die Bestimmung der gesamten Biomasse (X) in einem Bioprozess erfolgt nach dem Stand der Technik über eine manuelle Probenahme aus der Kulturbrühe, deren Aufarbeitung und der anschließenden gravimetrischen Messung im getrockneten Zustand. Dieses Messverfahren ist nicht nur fehleranfällig, sondern auch arbeitsaufwendig und das Ergebnis um mehrere Tage zeitverzögert. Weiterhin nachteilig ist, dass durch die Eingabe von X in der LPG auch das momentane Reaktorvolumen (V) bekannt sein muss. Da dem Bioreaktor Volumen (V) bspw. durch Substratzugabe, Probenahme etc. sowohl automatisiert als auch manuell zugeführt und abgelassen wird, ist auch die Bestimmung von V fehleranfällig und ungenau.

Um die LPG zur Echtzeit-Berechnung in einem Bioprozess nutzen zu können, muss in einem vorgelagerten Bioprozess X experimentell aufwendig bestimmt und mit einer weniger zeitverzögerten Messgröße korreliert werden. Bei dieser Messgröße handelt es sich nach dem Stand der Technik um die optische Dichte der Kulturbrühe. Die OD-X-Korrelation ist jedoch spezifisch und muss für jeden Mikroorganismus experimentell bestimmt werden.

Zum Stand der Technik gehört auch die Veröffentlichung von Krausch et al. (Literaturstelle [4]). Dieses Dokument zeigt in Figur 5 Messwerte für die Sauerstoff-Aufnahme-Rate OUR und die Biomasse zweier Kulturen von *E. coli,* angezogen in einer Kultivierungsplattform. In Tabelle 2 sind verschiedene Vergleichswerte für *E.* coli-Stämme wiedergegeben, darunter die Wachstumsrate $\mu_{max}$. Gemäß einem Luedeking-Piret-Modell werden auf Seite 7 zwei Gleichungen (3) und (4) für den Zusammenhang von OUR und Biomasse X angegeben. Sauerstoff und Kohlendioxid werden mit Gassensoren gemessen. Eine Auswertung der ermittelten Energieaufnahme nach einem mathematischen Modell, welches auf dem realen Verhalten der Auslaufgeschwindigkeit eines Behälters mit Zulauf und konstanter Auslauföffnung am Boden des Behälters in Abhängigkeit vom Druck der Flüssigkeitssäule basiert, wird aber dort nicht erwähnt.

**Aufgabe der Erfindung**

Der Erfindung liegt die Aufgabe zugrunde, die Nachteile der im Stand der Technik beschriebenen technischen Lösungen zu beseitigen.

**Die Lösung der Aufgabe**

Die Aufgabe wurde gemäß den Merkmalen der Patentansprüche gelöst. Zur Lösung der Problematik des im Stand der Technik beschriebenen populationsbasierten Wachstumsmodells (LPG), welches zwingend die Anfangsbiomasse (X) benötigt, wurde in dieser Erfindung erstmals ein Wachstumsmodell bereitgestellt, das mit einem der Biomasse proportionalen Energieanteil auskommt. Das erfindungsgemäße Modell nutzt die gleichen Modellparameter wie die LPG, benötigt jedoch keine Kenntnis über die Biomasse (X).

Das erfindungsgemäße Verfahren ist in den Patentansprüchen 1 bis 10 beschrieben worden. Anspruch 11 betrifft eine Anwendung dieses Verfahrens, Anspruch 12 ein Mittel zur Durchführung des Verfahrens und die Ansprüche 13 und 14 definieren ein universelles mathematisches Modell zur Beschreibung von Wachstumsprozessen in Kultivierungsvorrichtungen für Mikroorganismen.

**Technischer Hintergrund der Erfindung**

Die Erfindung beruht darauf, dass das Zellwachstum in einem Bioprozess durch ein neues Wachstumsmodell beschrieben wurde, indem das Zellwachstum auf die realen Verhältnisse, die sich in einem Regenfass abspielen, modellhaft übertragen wurde. Anstelle eines Regenfasses kann selbstverständlich jedes andere Fass mit einer beliebigen Flüssigkeit verwendet werden.

Der Vergleich mit einem Regenfass soll nun näher erläutert werden.

## Gedanken zur Herleitung eines neuen Wachstumsmodells

Da die spezifische Wachstumsrate (μ) per Definition die Zunahme der Biomasse (X) pro Zeiteinheit, bezogen auf die zu Beginn des Zeitintervalls (Δt) vorhandene Biomasse (X) ist, kann μ wie folgt berechnet werden:

$$\mu = \frac{d\,X}{dt} * \frac{1}{X} \qquad \text{Gleichung 6}$$

Aus der Proportionalität von EURm zur Biomasse (X), folgt:

$$\mu = \frac{d\,EURm}{dt} * \frac{1}{EURm} \qquad \text{Gleichung 7}$$

Die Herausforderung an das neue Wachstumsmodell liegt darin, die Energieanteile dEURm und EURm ohne Kenntnis der Biomasse (X) und somit über ein neues Berechnungsverfahren als die Gleichungen 4 und 5 zu ermitteln. Das neue Wachstumsmodell zur neuartigen Berechnung der Energieanteile ist bildlich abgeleitet aus der Funktion eines Regenfasses und wird im nachfolgenden Abschnitt näher beschrieben.

### Wie funktioniert ein Regenfass

Zum besseren Verständnis eines Wachstumsprozesses möchten wir zunächst die Funktion eines Regenfasses vorstellen. Im Garten steht ein Regenfass und, wenn es regnet, läuft Wasser vom Hausdach in das Regenfass, welches sich entsprechend der Regenmenge mehr oder weniger schnell füllt. An Tagen ohne Niederschlag gehe ich mit meiner Gießkanne zum Regenfass und fülle Wasser ab. Jetzt kommt ein wichtiges Phänomen ins Spiel, das jeder kennt. Ist das Regenfass bis oben gefüllt, so läuft viel Wasser aus dem Wasserhahn in meine Gießkanne. Ist das Regenfass jedoch fast leer, so läuft das Wasser nur noch sehr langsam ab und es dauert lange bis die Gießkanne voll ist. So kann festgestellt werden, die Menge Wasser, die in einem Zeitintervall aus dem Regenfass abfließt, ist vom Füllstand abhängig. So ist es überall in der Natur: Ein heißer Stein, der abkühlt, ein Regenfass, welches ausläuft, das Wachstum von Zellen. Alles geschieht nach der gleichen natürlichen Exponentialfunktion (e-Funktion).

### Interpretation eines Regenfasses als Wachstumsmodell

Wir betrachten nun die Regentropfen als Änderung der Energie, die das Regenfass befüllt. Der Inhalt des Regenfasses ist die Energie, die zur Zellteilung notwendig ist. Die Menge, die aus dem Regenfass herausläuft, betrachten wir als Energie, die zur Unterhaltung der gerade entstandenen neuen Zellen nötig ist.

Aus der abstrakten Betrachtung heraus wissen wir, dass die Änderung der Energie auch eine Änderung der Biomasse (X) zur Folge hat. Wachstum beginnt also mit der Änderung der Energie.

## Funktionsweise des Regenfassmodells (RFM)

Wir haben dieses Energieäquivalente-basierte Wachstumsmodell nach einem Regenfass benannt, weil es in anschaulicher Weise den mathematischen Zusammenhang zwischen Zell-Wachstum und Zell-Erhaltung über das Energieäquivalent in einer Kultivierungsvorrichtung erklärt.

Die folgenden Beispiele zeigen in unterschiedlicher Darstellung immer das gleiche Regenfassmodell (RFM). Das Eingangssignal $EUR_{(t)}$ ist immer das durch vorzugsweise Sensoren ermittelte Energieäquivalent der Energieaufnahme zu einem bestimmten Zeitpunkt (t).

Ziel dieses Modells ist die Bestimmung der Energie-Komponenten EURy, EURm und dEURm, um daraus die spezifische Wachstumsrate der Biomasse (μ) zu berechnen.

### Die Ausgangsgleichungen

$$EUR = EURy + EURm \qquad \text{(Gleichung 3 Summengleichung aus der Literatur)}$$

$$\mu = \frac{d\,EURm}{dt} * \frac{1}{EURm} \qquad \text{(Gleichung 7 folgt aus Gleichung 6, da X proportional EURm)}$$

Das Regenfassmodell kann erstmals die Komponenten dEURm und EURm allein aus der aufgenommenen Energie (EUR) mit Hilfe des konstanten Quotienten (m/y) berechnen. Dabei ist (m/y) das Verhältnis aus Biomasse-Erhaltungskoeffizient (m) und wahrer-Biomasse-Energie-Ausbeutekoeffizient (y) und symbolisiert die Abflussöffnung des Regenfasses.

(I) Bildhafte Erklärung in einfachen Worten

Die Änderung des Eingangssignals von $EUR_{(t)}$, also $dEUR_{(t)}$, befüllt das Regenfass. Gleichzeitig besitzt das Regenfass eine Auslauföffnung mit der Eigenschaft (m/y). Der Auslauf ($dEURm_{(t)}$ berechnet sich aus dem Füllstand * (m/y). Der Füllstand hat sich nun verringert und heißt jetzt $EURy_{(t)}$. Figur 1 zeigt eine schematische Darstellung des RFM (umrandet) mit den benötigten Sensoren als Auswerteeinheit einer Kultivierungsvorrichtung zur Beschreibung eines Wachstumsprozesses. In diesem Beispiel wird das Energieäquivalent im Gas-Zustrom und im Abgas ermittelt.

Figur 2 zeigt einen Signalflussplan des RFM zur Beschreibung eines Wachstumsprozesses in einer Kultivierungsvorrichtung. Das Eingangssignal $EUR_{(t)}$ entspricht dem, aus den Sensoren, ermittelten Energieäquivalent. Das Differentialglied berechnet die Änderung des Eingangssignals $EUR_{(t)}$ und gibt diese Änderung ($dEUR_{(t)}$) an das Integralglied weiter.

Im Integralglied sammelt sich das Ausgangssignal des Differentialglieds ($dEUR_{(t)}$) zum bildhaft gesprochenen Füllstand des Regenfasses. Über das Proportionalglied fließt eine dem Füllstand proportionale Größe mit dem Faktor (m/y) ab. Das Produkt entspricht $dEURm_{(t)}$, welches wiederum vom Eingangssignal des Integralgliedes subtrahiert wird. Der reduzierte Füllstand des Regenfasses heißt nun $EURy_{(t)}$. Über das Summierglied berechnet sich $EURm_{(t)}$ als Differenz zwischen $EUR_{(t)}$ und $EURy_{(t)}$. Die Biomasse (X) ist eine zu EURm(t) proportionale Größe.

(II) Mathematisch ausführliche Erklärung mit zeitdiskreten Gleichungen

(A) Das Eingangssignal $EUR_{(t)}$ ist das zyklisch durch die Sensoren ermittelte Energieäquivalent

(B) Die zyklische Änderung von $EUR_{(t)}$ (Ableitung nach der Zeit)

$$\mathrm{dEUR_{(t)}} = \mathrm{EUR_{(t)}} - \mathrm{EUR_{(t-1)}}$$

(C) Der Füllstand(t) steigt mit jedem Zyklus um $dEUR_{(t)}$.

$$\mathrm{Füllstand_{(t)}} = \mathrm{Füllstand_{(t-1)}} + \mathrm{dEUR_{(t)}} \qquad \textit{zyklische Summe von dEUR}$$

(D) Die zyklisch abfließende Menge des Füllstands entspricht $dEURm_{(t)}$.

$$\mathrm{dEURm_{(t)}} = \mathrm{Füllstand_{(t)}} * (\mathrm{m/y}) \qquad \textit{Erhaltungsenergie der gerade entstandenen neuen Zellen}$$

(E) Der $Füllstand_{(t)}$ ist nun um die abgeflossene Menge $dEURm_{(t)}$ reduziert.
Der reduzierte $Füllstand_{(t)}$ heißt jetzt $EURy_{(t)}$.

$$\mathrm{EURy_{(t)}} = \mathrm{Füllstand_{(t)}} - \mathrm{dEURm_{(t)}} \qquad \textit{Energieanteil zur Zellteilung}$$

(F) Die Differenz aus dem Sensorwert ($EUR_{(t)}$) und dem reduzierten Füllstand (EURy(t)) entspricht $EURm_{(t)}$.

$$\mathrm{EURm_{(t)}} = \mathrm{EUR_{(t)}} - \mathrm{EURy_{(t)}} \qquad \textit{Erhaltungsenergie aller lebender Zellen}$$

Die Berechnung (A) bis (F) ist für diesen Zyklus abgeschlossen und μ kann über die Gleichung 7 ermittelt werden. Mit einem neuen Sensorwert ($EUR_{(t)}$) beginnt die Berechnung wieder von vorne.

(III) Mathematisch prägnante Erklärung mit zeitdiskreten Gleichungen

$EUR_{(t)}$, zyklisch durch die Sensoren ermitteltes Energieäquivalent bzw. Eingangssignal.

$$dEUR_{(t)} = EUR_{(t)} - EUR_{(t-1)} \qquad \textit{Ableitung des Eingangssignals}$$

$$EURy_{(t)} = EURy_{(t-1)} + dEUR_{(t)} * (1 - (m/y)) \qquad \textit{Füllstand minus abgeflossene Menge}$$

$$dEURm_{(t)} = EURy_{(t-1)} + dEUR_{(t)} * (m/y) \qquad \textit{abgeflossene Menge}$$

$$EURm_{(t)} = EUR_{(t)} - EURy_{(t)} \qquad \textit{Anwendung der Summengleichung}$$

(IV) Stabilitätskriterien

Das RFM zeigt selbstkorrigierende Eigenschaften hinsichtlich des Füllstandes. Dazu folgendes bildhaftes Beispiel:

Auf einer Wiese stehen zehn gleiche Regefässer. Alle Regenfässer sind unterschiedlich gefüllt. Alle Regenfässer haben aber auch den gleichen geöffneten Ablasshahn.

**Versuch 1:** (triviale Lösung)

Es regnet nicht mehr und nach einer ausreichend langen Zeit sind alle Fässer leer, Sie haben also alle den gleichen Füllstand.

**Versuch 2:**

Es regnet mit konstantem Niederschlag. Aus den Fässern die gut gefüllt sind läuft auch viel Wasser heraus. Aus Fässern, die fast leer sind, läuft wenig Wasser hinaus und der Regen füllt das Fass weiter auf. Für alle Fässer kann gesagt werden, dass sich mit der Füllhöhe ein Gleichgewicht zur Niederschlagsmenge einstellt. Nach ausreichend langer Zeit sind alle Fässer gleich gefüllt, ungeachtet des Anfangsfüllstandes.

Bezogen auf das RFM bedeutet dies, dass mögliche Störungen der Sensorwerte zwar den Füllstand des Regenfasses beeinflussen, dieser sich aber schnell wieder selbst korrigiert.

(V) Methoden zur Bestimmung des Quotienten (m/y)

(A) Literaturwerte: Es gibt Tabellen aus denen (m/y) entnommen werden kann.

(B) Messung: Das RFM kann mit jedem beliebigen Schätzwert für (m/y) gestartet werden, wobei der Quotient (m/y) durch eine einzige Probe (Referenzmessung von $\mu$) auf den exakten Wert korrigiert werden kann. Diese Korrektur erfolgt über ein numerisches Verfahren.

(C) Messung (Stand der Technik): Die Parameter y und m können experimentell über die Geradengleichung des Luedeking-Piret-Modells bestimmt werden. Dazu sind mehrere zeitgleiche Referenzmessungen der Biomasse (X) und $\mu$ notwendig. Dieses Verfahren ist arbeitsaufwendig und fehleranfällig.

Bei einer Referenzmessung wird eine Probe aus der Kulturbrühe der Kultivierungsvorrichtung entnommen und deren Wert über ein Stand der Technik Verfahren ermittelt.

Die Erfindung soll nachfolgend anhand eines Ausführungsbeispiels näher erläutert werden, ohne die Erfindung auf dieses Beispiel zu beschränken.

**Ausführungsbeispiel des Regenfassmodells (RFM)**

Nachfolgend wird ein simuliertes Ausführungsbeispiel des RFM zur Ermittlung der spezifischen Wachstumsrate

(μ) in stark vereinfachter Form mit zehn Messzyklen beschrieben. Die Zahlenwerte der Energie-Anteile wurden aus dem Eingangssignal ($EUR_{(t)}$) über die Gleichungen des RFM aus Kapitel III berechnet. Die spezifische Wachstumsrate (μ) wurde aus den EnergieAnteilen über die Gleichung 7 berechnet. In diesem Ausführungsbeispiel werden drei unterschiedliche Phasen entsprechend dem Eingangssignal ($EUR_{(t)}$) beschrieben. Der Quotient (m/y) wird auf 0,2 festgelegt.

**Phase 1:**

$EUR_{(t)}$ ist konstant, aber auch nicht null, das Regenfass ist leer und wird nicht befüllt. Dies bedeutet: es findet kein Wachstum statt. Es existiert aber eine Biomasse (X) unbekannter Größe, welche eine Energie zur Erhaltung benötigt. In dieser Zeit gilt: $EUR_{(t)}$ = EURm(t) und EURy(t) = 0

**Phase 2:**

$EUR_{(t)}$ nimmt zyklisch um den gleichen Betrag ($dEUR_{(t)}$) zu. Das Regenfass beginnt sich zu füllen, während gleichzeitig der Füllstand, um den Faktor (m/y) proportional zum Füllstand sinkt. Ab jetzt beginnt ein Wachstum der Biomasse (X).

**Phase 3:**

$EUR_{(t)}$ ist konstant aber betragsmäßig größer als in Phase 1. Das Regenfass ist immer noch zu einem großen Teil befüllt, es kommt aber nichts Weiteres hinzu, sondern läuft langsam ab. In dieser Zeit werden weiterhin neue Zellen gebildet bis der Füllstand des Regenfasses aufgebraucht ist. Der Betrag von EURm(t) nähert sich asymptotisch $EUR_{(t)}$ an, bis das Regenfass vollständig entleert ist.

Tab. 1: Ausführungsbeispiel des RFM inklusive der Bestimmung der spezifischen Wachstumsrate (μ) mit Zahlenwerten.

| Phase | Messzyklus | EUR | dEUR | EURy | dEURm | EURm | μ |
|---|---|---|---|---|---|---|---|
| 1 | 1 | 1 | 0 | 0,000 | 0,000 | 1,000 | 0,000 |
| 1 | 2 | 1 | 0 | 0,000 | 0,000 | 1,000 | 0,000 |
| 2 | 3 | 2 | 1 | 0,800 | 0,200 | 1,200 | 0,167 |
| 2 | 4 | 3 | 1 | 1,440 | 0,360 | 1,560 | 0,231 |
| 2 | 5 | 4 | 1 | 1,952 | 0,488 | 2,048 | 0,238 |
| 3 | 6 | 4 | 0 | 1,562 | 0,390 | 2,438 | 0,160 |
| 3 | 7 | 4 | 0 | 1,249 | 0,312 | 2,751 | 0,114 |
| 3 | 8 | 4 | 0 | 0,999 | 0,250 | 3,001 | 0,083 |
| 3 | 9 | 4 | 0 | 0,800 | 0,200 | 3,200 | 0,062 |
| 3 | 10 | 4 | 0 | 0,640 | 0,160 | 3,360 | 0,048 |

In Figur 3 ist das aus den Sensoren ermittelte Eingangssignal (EUR) über die Messzyklen abgebildet.

Figur 4 zeigt die aus dem RFM berechneten Energieanteile zur Zellteilung (EURy) und zur Zellerhaltung der bestehenden Zellen (EURm) über die Messzyklen. Die Summe beider Energieanteile ergeben das Eingangssignal (EUR).

Figur 5 zeigt die über das RFM ermittelte Zielgröße (μ) über die Messzyklen.

In Figur 6 ist der Kurvenverlauf eines ähnlichen Ausführungsbeispiel in höherer Auflösung mit 1500 Messzyklen statt 10 Messzyklen dargestellt.

## LPG und RFM im Vergleich

In den nachfolgenden Abschnitten wird das Literaturmodell (LPG) und das RFM auf unterschiedliche Weise miteinander verglichen.

**Die unterschiedlichen mathematischen Ansätze**

Im nachfolgenden Abschnitt soll der Unterschied der Wachstumsmodelle LPG und RFM in einer Gegenüberstellung der jeweiligen Gleichungen verdeutlicht werden.

Beide Ansätze zur Berechnung der spezifischen Wachstumsrate ($\mu$) beruhen zunächst auf derselben Summengleichung der Energieanteile:

$$\mathbf{EUR = EURy + EURm} \qquad \text{Gleichung 3 (Summengleichung)}$$

Die LPG (Literaturmodell) und das neue RFM berechnen die einzelnen Terme (EURy und EURm) auf unterschiedliche Weise. Die LPG auf Basis eines Wachstumsmodells der Population und das RFM auf dem realen Verhalten der Auslaufgeschwindigkeit eines Wasserfasses mit konstanter Auslauföffnung am Boden des Fasses in Abhängigkeit vom hydrostatischen Druck der Wassersäule.

Tab. 2: Aufführung der Gleichungen zur unterschiedlichen Berechnung der Zielgröße (spezifische Wachstumsrate $\mu$) in einer Kultivierungsvorrichtung über das RFM und das Literatur-Modell (LPG). Das RFM und die LPG liefern das gleiche Ergebnis für $\mu$.

| Berechnung von: | LPG | RFM |
|---|---|---|
| Änderung der Energie | wird nicht benötigt | $dEUR_{(t)} = EUR_{(t)} - EUR_{(t-1)}$ |
| Energie zur Zellteilung (aller Zellen) | $EURy_{(t)} = \mu_{(t)} * X_{(t)} * y$ | $EURy_{(t)} = EURy_{(t-1)} + dEUR_{(t)} * (1 - (m/y))$ |
| Energie zur Zellerhaltung (der im Zyklus neu entstandenen Zellen) | wird nicht benötigt | $dEURm_{(t)} = EURy_{(t-1)} + dEUR_{(t)} * (m/y)$ |
| Energie zur Zellerhaltung (aller Zellen) | $EURm_{(t)} = X(t) * m$ | $EURm_{(t)} = EUR_{(t)} - EURy_{(t)}$ |
| Biomasse | $X_{(t)} = X_{(t-1)} + X_{(t)} * \mu_{(t)}$ Gleichbedeutend mit: $X_{(t)} = X_{(t-1)} + X_{(t)} * \mu_{(t)}$ | wird nicht benötigt, kann aber über das Verhältnis X ~ EURm berechnet werden. (der Proportionalitätsfaktor ist m) |
| Spez. Wachstumsrate | $\mu_{(t)} = \frac{EUR_{(t)}/X_{(t)}}{y} - \frac{m}{y}$ | $\mu = \frac{d\,EURm}{dt} * \frac{1}{EURm}$ |

**Differentialgleichungen**

Nachfolgend werden die Differentialgleichungen der LPG und des RFM gegenübergestellt:

Tab. 3: Die Differentialgleichung der LPG (links) und des RFM (rechts).

| LPG | RFM |
|---|---|
| $EUR = y * \frac{dx}{dt} + m * X$ | $EUR = EURy + \left(\frac{m}{y}\right) * \frac{d\,EURy}{dt}$ |

Bei der LPG ist der Sammler (das Integral) die Biomasse (X). Ohne diesen Sammler hat die Gleichung keinen Bestand. Aus diesem Grund ist auch der Startwert ($X_0$) obligatorisch (siehe e-Funktion Gleichung 1). Bei dem RFM ist der Sammler (das Integral) der Füllstand bzw. EURy. Die Biomasse (X) kommt in dieser Differentialgleichung nicht vor.

**Das Anfangswertproblem**

Ein Anfangswertproblem liegt vor, wenn eine Differentialgleichung zu Beginn durch einen bestimmten Punkt verlaufen muss.

Bei der LPG muss deswegen für Gleichung 1 (Wachstumsmodell der Population) ein Anfangswert der Biomasse ($X_0$) festgelegt werden. Ohne $X_0$ kann es kein Biomasse-Wachstum geben.

Bei dem RFM kann, wenn zum Startzeitpunkt $\mu$ deutlich größer null ist, ein Anfangsfüllstand des Regenfasses bzw. EURy festgelegt werden. Dieser Anfangswert kann bspw. über eine Referenzmessung von $\mu$ berechnet werden. Ist das Biomasse-Wachstum, also $\mu$, zum Startzeitpunkt noch sehr klein oder null, so ist der Füllstand des Regenfasses leer und damit das Anfangswertproblem gelöst. Ein Wachstum ist auch bei anfänglich leerem Regenfass möglich.

Wird der Anfangsfüllstand generell nicht beachtet, so korrigiert sich dieser nach ausreichend langer Zeit selbst, siehe Abschnitt "Stabilitätskriterium".

**Behauptung und Beweisführung**

Wir behaupten, dass die Berechnung der Zielgröße (spezifische Wachstumsrate $\mu$) sowie die Energieanteile (EURy) und (EURm) über die LPG und das RFM die gleichen Ergebnisse über unterschiedliche Rechenwege liefern. In den nachfolgenden Abschnitten A), B) und C) werden die Beweise für diese Behauptung geführt. Nachfolgend sind, die für die Beweisführung notwendigen Gleichungen 1 bis 7, nochmal aufgeführt:

$$X_{(t)} = X_{(0)} * e^{\mu_{(t)} * \Delta t} \qquad \text{Gleichung 1}$$

$$\mu_{(t)} = \frac{EUR_{(t)}/X_{(t)}}{y} - \frac{m}{y} \qquad \text{Gleichung 2 (LPG)}$$

$$EUR = EURy + EURm \qquad \text{Gleichung 3 Literatur}$$

$$EURy_{(t)} = \mu_{(t)} * X_{(t)} * y \qquad \text{Gleichung 4 LPG}$$

$$EURm_{(t)} = X_{(t)} * m \qquad \text{Gleichung 5 LPG}$$

$$\mu = \frac{dX}{dt} * \frac{1}{X} \qquad \text{Gleichung 6 Definition Literatur}$$

$$\mu = \frac{d\,EURm}{dt} * \frac{1}{EURm} \qquad \text{Gleichung 7 RFM}$$

**A) Allgemeingültiger Beweis für alle Wachstumsphasen**

Unter der Annahme, dass EURy und EURm bekannt seien und getrennt voneinander ermittelt werden könnten, kommt man zur folgenden Gegenüberstellung:

Tab. 4: Gleichungen der LPG (links) und des RFM (rechts).

| LPG | RFM |
|---|---|
| (a) Gleichung 6, Definition der spez. Wachstumsrate $\mu = \frac{dX}{dt} * \frac{1}{X}$ | (a) Gleichung 7 $\mu = \frac{d\,EURm}{dt} * \frac{1}{EURm}$ |
| (b) Gleichung 4 dividiert durch Gleichung 5 $\frac{EURy}{EURm} = \frac{\mu * X * y}{X * m}$ | (b) Abfließende Menge proportional dem Füllstand |
| (c) gekürzt $\frac{EURy}{EURm} = \mu * \frac{1}{(m/y)}$ | dEURm = EURy * (m/y)<br>(c) b in a eingesetzt |

(fortgesetzt)

| LPG | RFM |
|---|---|
| (d) nach μ umgestellt<br>$\mu = \frac{EURy}{EURm} * (m/y)$ | $\frac{EURy}{EURm} * (m/y) = \mu$<br>(d) nach μ umgestellt<br>$\mu = \frac{EURy}{EURm} * (m/y)$ |

Beweis: die LPG und das RFM können μ theoretisch über dieselbe Ausgangsgleichung berechnen. Die Problematik der LPG gegenüber dem RFM liegt jedoch darin, dass EURy und EURm über die Biomasse (X) und μ (siehe Gleichungen 4 und 5) bestimmt werden. Das heißt, die LPG kann weder EURy noch EURm noch den Quotienten (EURy/EURm) allein aus dem Eingangssignal EUR bestimmen.

**B) Beispiel, wenn kein Wachstum stattfindet (EUR = konstant)**

Nach ausreichend langer Zeit gilt: Es findet kein Wachstum mehr statt, die aufgenommene Energie wird ausschließlich zur Erhaltung der bestehenden Zellen genutzt, somit ist EUR konstant.

[0060] Tab. 5: Gleichungen der LPG (links) und des RFM (rechts). Beweis: EUR entspricht EURm sowohl bei der LPG als auch bei dem RFM, wenn kein Wachstum mehr stattfindet.

| LPG | RFM |
|---|---|
| *(a) Ausgangsbedingung, es entstehen keine neuen Zellen*<br>EURy = 0<br><br>*(b) Gleichung 3, Summengleichung*<br>EUR = EURy + EURm<br>*(c) weil EURy = 0*<br>EUR = EURm<br>*(d) Energie zur Erhaltung der bestehenden Zellen*<br>EUR = EURm | *(a) Ausgangsbedingung, das Regenfass ist leer*<br>EURy = 0<br>*(b) Gleichung 3, Summengleichung*<br>EUR = EURy + EURm<br>*(c) weil EURy = 0*<br>EUR = EURm<br>*(d) Energie zur Erhaltung der bestehenden Zellen*<br>EUR = EURm |

C) **Beispiel für lineare Biomasse-Zunahme**

Die lineare Zunahme der Biomasse (X) bedeutet, dass zu jedem Zeitintervall gleich viele neue Zellen hinzukommen. Wenn EURy ein Maß für neu entstehende Zellen ist und das Wachstum linear ist, so folgt: EURy = const und die Ableitung dEURy = 0. Für das RFM bedeutet dies, dass sich ein konstanter Füllstandspegel einstellt, da genau so viel aus dem Regenfass hinausläuft, wie im gleichen Zeitraum hineinkommt.

Tab. 6: Gleichungen der LPG (links) und des RFM (rechts).

| LPG | RFM |
|---|---|
| *(a) Ausgangsbedingung*<br>EURy = const<br><br>*(b) Ableitung von (a)*<br>dEURy = 0<br><br>*(c) Die Summengleichung abgeleitet*<br>dEUR = dEURy + dEURm<br><br>*(d) ergibt sich aus (b), weil dEURy = 0* | |

(fortgesetzt)

| LPG | RFM |
|---|---|
| dEUR = dEURm | |
| *(e) Gleichung 5*<br>EURm = X * m<br>*(f) Ableitung von Gleichung 5*<br>$$\mathrm{dEURm} = \mathrm{m} * \frac{\mathrm{dX}}{\mathrm{dt}}$$<br>*(g) umgestellt nach dx/dt*<br>$$\frac{\mathrm{dX}}{\mathrm{dt}} = \frac{\mathrm{dEURm}}{\mathrm{m}}$$<br>*(h) Gleichung 4*<br>EURy = μ * X * y<br>*(i) für p wird Gleichung 6 in (h) eingesetzt*<br>$$\mathrm{EURy} = \mathrm{y} * \frac{\mathrm{dX}}{\mathrm{dt}}$$<br>*(j) (g) in (i) eingesetzt*<br>$$\mathrm{EURy} = \mathrm{y} * \frac{\mathrm{dEURm}}{\mathrm{m}}$$<br>*(k) umgestellt*<br>$$\mathrm{EURy} = \frac{\mathrm{dEURm}}{(\mathrm{m/y})}$$ | *(e) die abfließende Menge ist proportional dem Füllstand, siehe Herleitung RFM*<br>dEURm = EURy * (m/y)<br><br>*(k) umgestellt*<br>$$\mathrm{EURy} = \frac{\mathrm{dEURm}}{(\mathrm{m/y})}$$ |
| *(l) Anwendung von (d) dEUR = dEURm*<br>$$\mathrm{EURy} = \frac{\mathrm{dEUR}}{(\mathrm{m/y})}$$ | *(l) Anwendung von (d) dEUR = dEURm*<br>$$\mathrm{EURy} = \frac{\mathrm{dEUR}}{(\mathrm{m/y})}$$ |

Beweis: In beiden Modellen berechnet sich die Energie zur Zellteilung über die Gleichung EURy = dEUR/(m/y).

**Beispiel mit realen Daten**

Im Labor der Hochschule Biberach wurde die $O_2$-Konzentration im Gas-Zustrom und Abgas eines 41-stündigen Bioprozesses zur Kultivierung von Leuconostoc mesenteroides mit Hilfe eines Abgassensor-Prototyps aufgenommen und über die LPG und das RFM ausgewertet. In Figur 7 und 8 sind jeweils die Kurvenverläufe der spezifischen Wachstumsrate, des OUR, des OURy, des OURm und der Biomasse in verschiedenen Linienformen dargestellt. In diesem Beispiel wurde die Abkürzung OUR (Sauerstoff-Aufnahme-Rate) anstelle von EUR gewählt, da es sich bei der Energie proportionalen Größe um die Anzahl der $O_2$-Moleküle handelt. Die Figur 7 zeigt die Auswertung der Sensordaten nach der LPG. Die notwendigen Prozessparameter waren: $X_0$ = 1,4 g, y = 0.024 mol/g und m = 0,0018 mol/(g*h). Die Figur 8 zeigt die Auswertung der Sensordaten nach dem RFM. Die notwendigen Prozessparameter waren: (m/y) = 0,075 1/h. Auf eine Anfangs-Füllstandskorrektur über die Bestimmung von μ zum Startzeitpunkt (siehe Anfangswertproblem) wurde absichtlich verzichtet. So soll gezeigt werden, dass die Kurvenverläufe von μ, ermitteltet über die LPG und das RFM bereits nach kurzer Zeit deckungsgleich sind (siehe Stabilitätskriterien). Zusammenfassend kann gesagt werden, dass das RFM auch für reale Daten das gleiche Ergebnis für μ, wie über die LPG berechnet, liefert. Das RFM zeigt somit auch in der Praxis keine Nachteile gegenüber der LPG.

**Bekannte mathematische Näherungen**

Wie zuvor beschrieben zeigt die LPG aufgrund der Biomasse (X) als Modellparameter die Schwierigkeiten, welche (A) die Einfachheit der Bedienung für den Endverbraucher und (B) die Genauigkeit der Zielgrößen-Berechnung (μ) beeinflussen. Aus diesem Grund bedient sich die Literatur mathematischer Näherungen der LPG. Diese mathematischen Näherungen sind Vereinfachungen der LPG, welche zwar weniger Modellparameter benötigen, jedoch nur in bestimmten Wachstumsphasen Gültigkeit besitzen oder anderweitig Nachteile gegenüber der LPG aufzeigen. Nachfolgend werden die bekannten mathematischen Näherungen der LPG aus der Literatur und das RFM mit der LPG verglichen.

Tab. 7: Auflistung der Vor- und Nachteile der LPG, mathematischen Näherungen der LPG und des RFM.

| **Mathematisches Modell** | **Vorteil** | **Nachteil** |
|---|---|---|
| LPG | - Gültigkeit in allen Wachstumsphasen | - die Prozessparameter m und y müssen einzeln bestimmt werden<br>- Bestimmung der Biomasse (X) notwendig |
| Math. Näherung der LPG nach "Practical Solutions for Specific Growth Rate Control Systems in Industrial Bioreactors" aus dem Jahr 2019, Gleichung 8<br>$\mu = \frac{dEUR}{dt} * \frac{1}{EUR}$ | - keine Verwendung der Biomasse (X)<br>- keine Verwendung der Modellparameter m und y | - Nur in bestimmten Wachstumsphasen gültig (gültig, wenn $\mu$ = const.)<br>- schlechtes Signal-RauschVerhältnis |
| Math. Näherung der LPG nach "Practical Solutions for Specific Growth Rate Control Systems in Industrial Bioreactors" aus dem Jahr 2019, Gleichung 9<br>$\mu = \frac{EUR}{\sum EUR * dt}$ | - keine Verwendung der Biomasse (X)<br>- keine Verwendung der Modellparameter m und y | - Nur in bestimmten Wachstumsphasen gültig (gültig, wenn Biomasse-Wachstum exponentiell) |
| Math. Näherung der LPG nach "An oxygen-uptake-rate-based estimator of the specific growth rate in Escherichia coli BL21 strains cultivation process" aus dem Jahr 2021, Gleichung 10<br>$\mu = \frac{dEUR}{dt} * \frac{1}{EUR} - \frac{1}{\mu + m/y} * \frac{d\mu}{dt}$ | - keine Verwendung der Biomasse (X)<br>- die Prozessparameter m und y können als Quotient (m/y) verwendet werden | - Nur in bestimmten Wachstumsphasen gültig (gültig, wenn $\mu$ = const.)<br>- sehr schlechtes Signal-Rausch-Verhältnis |
| RFM aus dem Jahr 2024 | - Gültigkeit in allen Wachstumsphasen<br>- keine Verwendung der Biomasse (X)<br>- die Prozessparameter m und y können als Quotient (m/y) verwendet werden | Keine Nachteile gegenüber der LPG |

**Bestimmung der Energie proportionalen Messgröße (EUR)**

Die LPG und das neue Wachstumsmodell nutzen eine Energie proportionale Messgröße (EUR) als Eingangssignal zur Bestimmung der spezifischen Wachstumsrate ($\mu$) in einer Kultivierungsvorrichtung. Eine Energie proportionale Messgröße kann die Aufnahme von $O_2$, aber auch die Bildung von $CO_2$ oder jede andere, der Energie proportionalen Größe wie die Substrataufnahme oder die Produktbildung sein. Die nachfolgende Erläuterung ist Stand der Technik und dient zum besseren Verständnis.

Für die Messung von EUR in einer Kultivierungsvorrichtung wird in diesem Beispiel die Anzahl der aufgenommenen $O_2$-Moleküle als Energie proportionale Messgröße verwendet. Die dafür benötigten Sensoren sind:

A) ein Massendurchflusssensor für Gas
B) ein Gassensor, der die Konzentration eines bestimmten Gases in einem Gasgemisch identifiziert. Für dieses Beispiel wird $O_2$ gewählt.

Figur 0 zeigt eine Kultivierungsvorrichtung mit einer Messeinrichtung im Gas-Zustrom und im Abgas zur jeweiligen Bestimmung des Massendurchflusses für Gas und der $O_2$-Konzentration.

Das Produkt aus $O_2$-Konzentration und Gasfluss ist proportional der Anzahl der $O_2$-Moleküle.

Die Differenz aus der Anzahl der $O_2$-Moleküle im Gas-Zustrom und der Anzahl der $O_2$-Moleküle im Abgas ist proportional der momentan verbrauchten Energie. EUR ist also ein Äquivalent zur aufgenommenen Energie und wird wie folgt berechnet:

$$\mathbf{EUR \sim O2_{in} * Gasfluss_{in} - O2_{out} * Gasfluss_{out}}$$

## Zeitdiskrete Schreibweise

Kurze Erklärung der zeitdiskreten Schreibweise (Transformation nach Laplace)

Die Messwerterfassung und auch die Auswertung erfolgt in einem fest definierten, konstanten Zeitintervall (Δt). Alle Prozessparameter werden so normiert, dass sich als Zeitintervall der Wert "1" ergibt. Aus diesem Grund kann der Faktor "Zeit = 1" entfallen.

Z-Transformation: Grundlage für die zyklische Berechnung

$$y = \frac{dx}{dt} \quad \rightarrow y_{(t)} = X_{(t)} - X_{(t-1)} \quad \text{Der Differentialquotient}$$

$$y = \int_0^t X\,dt \quad \rightarrow y_{(t)} = y_{(t-1)} + X_{(t)} \quad \text{Das Integral}$$

$$y = Xo\ e^{\mu t} \quad \rightarrow y_{(t)} = y_{(t)} + X_{(t)} * \mu \quad \text{Die e-Funktion}$$

**Definition:**

Für den math. Ausdruck $y = \frac{dx}{dt}$ verwenden wir die Schreibweise *y* = *dx* oder y = *Δx* Unsere Schreibweise von *dx* ist gleichbedeutend mit $X_{(t)} - X_{(t-1)}$.

## Abkürzungsverzeichnis

| | |
|---|---|
| **FDA** | U.S. Food and Drug Administration |
| **-in und -out** | Endung zur Beschreibung von Gas-Zustrom und Abgas |
| **LPG** | Luedeking-Piret-Gleichung |
| **OD** | Optische Dichte |
| **OUR** | Sauerstoff-Aufnahme-Rate |
| **RFM** | Regenfassmodell |

## Formelzeichen

| Formelzeichen | Bedeutung | Einheit |
|---|---|---|
| **$CO_2$** | Kohlenstoffdioxid | [%] |
| **dEURm** | Energieaufnahme für die Erhaltung der gerade neugebildeten Zellen im Zeitintervall Δt | [J/h] |
| **EUR** | Energieaufnahme | [J] |
| **EURm** | Energieaufnahme für die Erhaltung der bestehenden und der neugebildeten Zellen | [J] |

(fortgesetzt)

| Formelzeichen | Bedeutung | Einheit |
|---|---|---|
| **EURy** | Energieaufnahme für die Zellteilung | [J] |
| **Füllstand** | Füllstand des Regenfasses | [J] |
| **(m/y)** | Quotient aus Biomasse-Erhaltungskoeffizient (m) und wahrer Biomasse-Energie-Ausbeutekoeffizient (y) | [1/h] |
| **$O_2$** | Sauerstoff | [%] |
| **t** | Zeitpunkt im Messzyklus | [h] |
| **V** | Reaktorvolumen | [L] |
| **X** | Biomasse oder Zellen | [g] |
| **$X_0$** | Anfängliche Biomasse | [g] |
| **$\Delta t$** | Zeitintervall | [h] |
| **$\mu$** | spezifische Wachstumsrate der Biomasse | [1/h] |

**Literaturverzeichnis**

[1] M. M. Silveira and M. A. B. Molina, "Indirect estimation of Bacillus thuringiensis var. israelensis biomass concentration using oxygen balance data," Brazilian Journal of Chemical Engineering, vol. 22, no. 4, pp. 495-500, 2005.

[2] D. W. Zabriskie and A. E. Humphrey, "Real-time estimation of aerobic batch fermentation biomass concentration by component balancing," AIChE Journal, vol. 24, no. 1, pp. 138-146, 1978.

[3] R. Luedeking and E. L. Piret, "A kinetic study of the lactic acid fermentation. Batch process at controlled pH," Journal of Biochemical and Microbiological Technology and Engineering, vol. 1, no. 4, pp. 393-412, 1959.

[4] Krausch et al. "Model-Based Characterization of E. coli Strains with Impaired Glucose Uptake", Bioengineering 2023, 10, 808, https: //doi.org/10.3390/bioengineering10070808

**Patentansprüche**

1. Verfahren zur Bestimmung der Wachstumsrate ($\mu$) in einer Kultivierungsvorrichtung für Mikroorganismen, umfassend:

   a) Messung eines Energie-Äquivalents im Gas-Zustrom der Kultivierungsvorrichtung und Messung eines Energie-Äquivalents im Abgas der Kultivierungsvorrichtung und - resultierend aus deren Differenz - die Ermittlung eines Äquivalents der in der Kultivierungsvorrichtung erfolgten Energieaufnahme (EUR) oder
   b) Messung der Konzentrationsänderung eines Energie-Äquivalents (Substrat-/Produktkonzentration) innerhalb der Kultivierungsvorrichtung und - resultierend aus deren zeitlichen Differenz - die Ermittlung eines Äquivalents in der Kultivierungsvorrichtung erfolgten Energieaufnahme (EUR) und
   c) Auswertung der ermittelten Energieaufnahme nach einem mathematischen Modell, welches auf dem realen Verhalten der Auslaufgeschwindigkeit eines Behälters mit Zulauf und konstanter Auslauföffnung am Boden des Behälters in Abhängigkeit vom Druck der Flüssigkeitssäule basiert,

   wobei die Bestimmung der Wachstumsrate ($\mu$) allein aus dem ermittelten Äquivalent der Energieaufnahme unter Anwendung des mathematischen Modells 1c) erfolgt und eine Kenntnis der Biomasse (X) als Startwert nicht erforderlich ist.

2. Verfahren nach Anspruch 1, **dadurch gekennzeichnet, dass** das mathematische Modell, auf dem realen Verhalten der Auslaufgeschwindigkeit eines Wasserfasses (Regenfassmodell - RFM) mit konstanter Auslauföffnung am Boden des Fasses in Abhängigkeit vom hydrostatischen Druck der Wassersäule basiert.

3. Verfahren nach Anspruch 1 oder 2, **dadurch gekennzeichnet, dass** als Energie-Äquivalent

i. im Gas-Zustrom und im Abgas der Kultivierungsvorrichtung die Sauerstoffkonzentration oder $CO_2$-Konzentration gemessen wird oder
ii. der Verbrauch eines Substrats oder die Bildung eines Produktes innerhalb der Kultivierungsvorrichtung gemessen wird.

**4.** Verfahren nach einem der Ansprüche 1 bis 3, **dadurch gekennzeichnet, dass** die Energieaufnahme (EUR) gemäß Anspruch 1a) oder 1b) sich aus der Energie, die für die Zellteilung benötigt wird (EURy) und aus der Energie, die für die Erhaltung aller Zellen, also der bestehenden und der neugebildeten Zellen, benötigt wird (EURm) gemäß Gleichung 3:

$$EUR = EURy + EURm$$

zusammensetzt, wobei EURm proportional der Biomasse (X) ist.

**5.** Verfahren nach einem der Ansprüche 1 bis 4, **dadurch gekennzeichnet, dass** eine gemessene zeitliche Änderung der Energieaufnahme von EUR zu einem Zeitpunkt t ($EUR_{(t)}$), also $dEUR_{(t)}$, im mathematischen Modell der Befüllung des Behälters gemäß Anspruch 1c), insbesondere der Befüllung des Regenfasses gemäß Anspruch 2, entspricht.

**6.** Verfahren nach einem der Ansprüche 1 bis 5, **dadurch gekennzeichnet, dass** die zeitliche Änderung der Energieaufnahme für die Erhaltung der gerade neugebildeten Zellen dEURm(t) im mathematischen Modell gemäß Anspruch 1c) einer zyklisch abfließenden Menge der Flüssigkeit am Auslauf des Behälters entspricht, die abhängig vom Füllstand des Behälters ist.

**7.** Verfahren nach Anspruch 6, **dadurch gekennzeichnet, dass** sich $dEURm_{(t)}$ aus dem Füllstand zum Zeitpunkt t multipliziert mit einer Konstanten (m/y) berechnen lässt.

**8.** Verfahren nach einem der Ansprüche 1 bis 6, **dadurch gekennzeichnet, dass** die momentane Energie, die für die Zellteilung benötigt wird ($EURy_{(t)}$), im mathematischen Modell dem verringerten Füllstand des Behälters entspricht.

**9.** Verfahren nach einem der Ansprüche 1 bis 8, **dadurch gekennzeichnet, dass** die Erhaltungsenergie aller lebender Zellen zu einem Zeitpunkt t ($EURm_{(t)}$) aus der gemessenen Energieaufnahme von EUR zu einem Zeitpunkt t $EUR_{(t)}$ abzüglich des gemäß Anspruch 8 ermittelten Wertes von $EURy_{(t)}$ bestimmt wird.

**10.** Verfahren nach einem der Ansprüche 1 bis 9, **dadurch gekennzeichnet, dass** sich die Wachstumsrate ($\mu$) in einer Kultivierungsvorrichtung für Mikroorganismen aus dem Äquivalent der in der Kultivierungsvorrichtung erfolgten Energieaufnahme (EUR) gemäß der Gleichung 4:

$$\mu = \frac{d\,EURm}{dt} * \frac{1}{EURm}$$

berechnet, wobei die Komponenten dEURm und EURm aus dem aufgenommenen Energie-Äquivalent (EUR) gemäß Anspruch 1a) oder 1b) unter Anwendung des mathematischen Modells gemäß Anspruch 1c) ermittelt werden.

**11.** Verwendung des Verfahrens nach einem der Ansprüche 1 bis 10 zur Ermittlung der Biomasse (X) in einer Kultivierungsvorrichtung für Mikroorganismen, da X proportional zu EURm ist.

**12.** Mittel zur Bestimmung der Wachstumsrate ($\mu$) in einer Kultivierungsvorrichtung für Mikroorganismen, umfassend:

- mindestens ein Messgerät zur Ermittlung der Gasmenge im Gas-Zustrom der Kultivierungsvorrichtung und
- mindestens ein Messgerät (Sensor) zur Ermittlung der Konzentrationsdifferenz mindestens eines Gases zwischen Gas-Zustrom-Konzentration und Abgas-Konzentration der Kultivierungsvorrichtung oder
- mindestens ein Messgerät (Sensor) zur Ermittlung der Konzentrationsänderung eines Substrats oder Produkts innerhalb der Kulturvorrichtung, welches auf die Energieaufnahme schließen lässt
- eine Auswerte-Einheit (Computer), auf der eine Software installiert ist, welche die vom Sensor übermittelten Daten nach einem mathematischen Modell auswertet, das auf dem realen Verhalten der Auslaufgeschwindigkeit eines Behälters mit Zulauf und konstanter Auslauföffnung am Boden des Behälters in Abhängigkeit vom Druck der Flüssigkeitssäule basiert.

**13.** Universelles mathematisches Modell zur Beschreibung von Wachstumsprozessen in Kultivierungsvorrichtungen für Mikroorganismen, insbesondere zur Ermittlung des Zusammenhangs zwischen Zell-Wachstum und Zell-Erhaltung, das gemessene Werte für Energieäquivalente so auswertet, als ob die Wachstumsprozesse einem realen Verhalten der Auslaufgeschwindigkeit eines Behälters mit Zulauf und konstanter Auslauföffnung am Boden des Behälters in Abhängigkeit vom Druck der Flüssigkeitssäule entsprechen.

**14.** Universelles mathematisches Modell nach Anspruch 13, **dadurch gekennzeichnet, dass** es sich um ein Regenfass-Modell handelt.

Figur 0

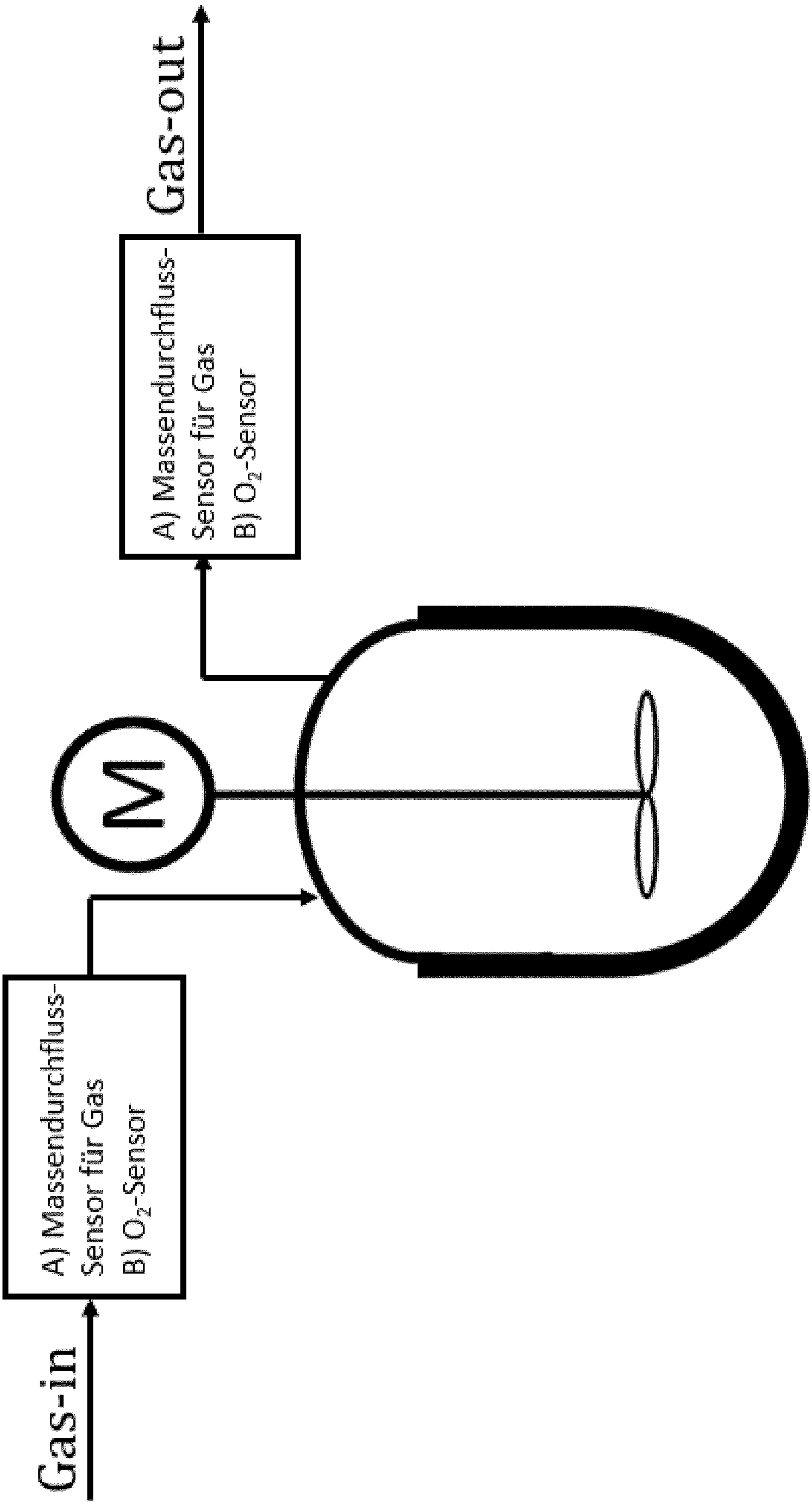
Gas-in
A) Massendurchfluss-Sensor für Gas
B) $O_2$-Sensor
M
A) Massendurchfluss-Sensor für Gas
B) $O_2$-Sensor
Gas-out

Figur 1

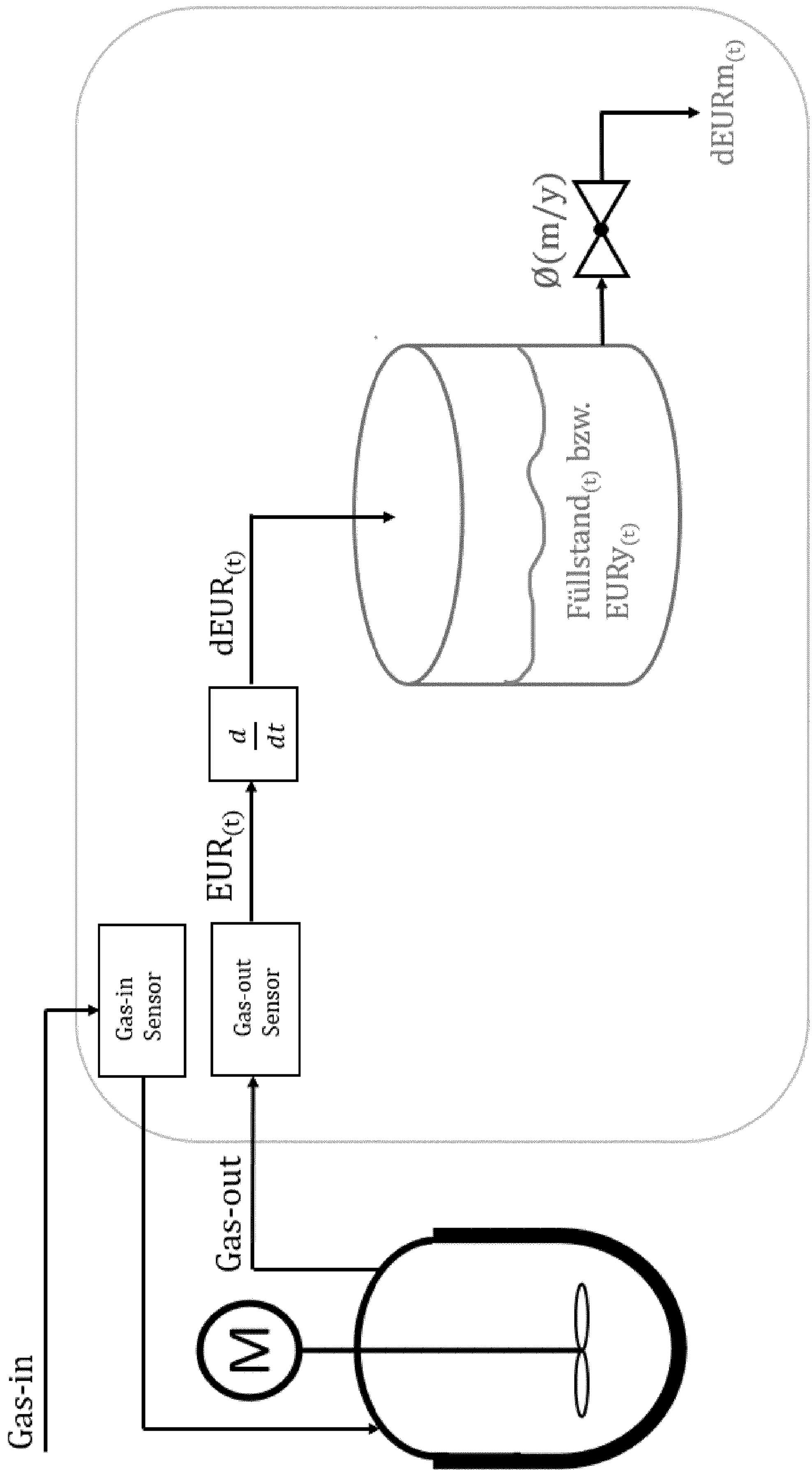
Gas-in
Gas-in Sensor
Gas-out Sensor
Gas-out
M
$EUR_{(t)}$
$\frac{d}{dt}$
$dEUR_{(t)}$
$Füllstand_{(t)}$ bzw. $EURy_{(t)}$
Ø(m/y)
$dEURm_{(t)}$

Figur 2

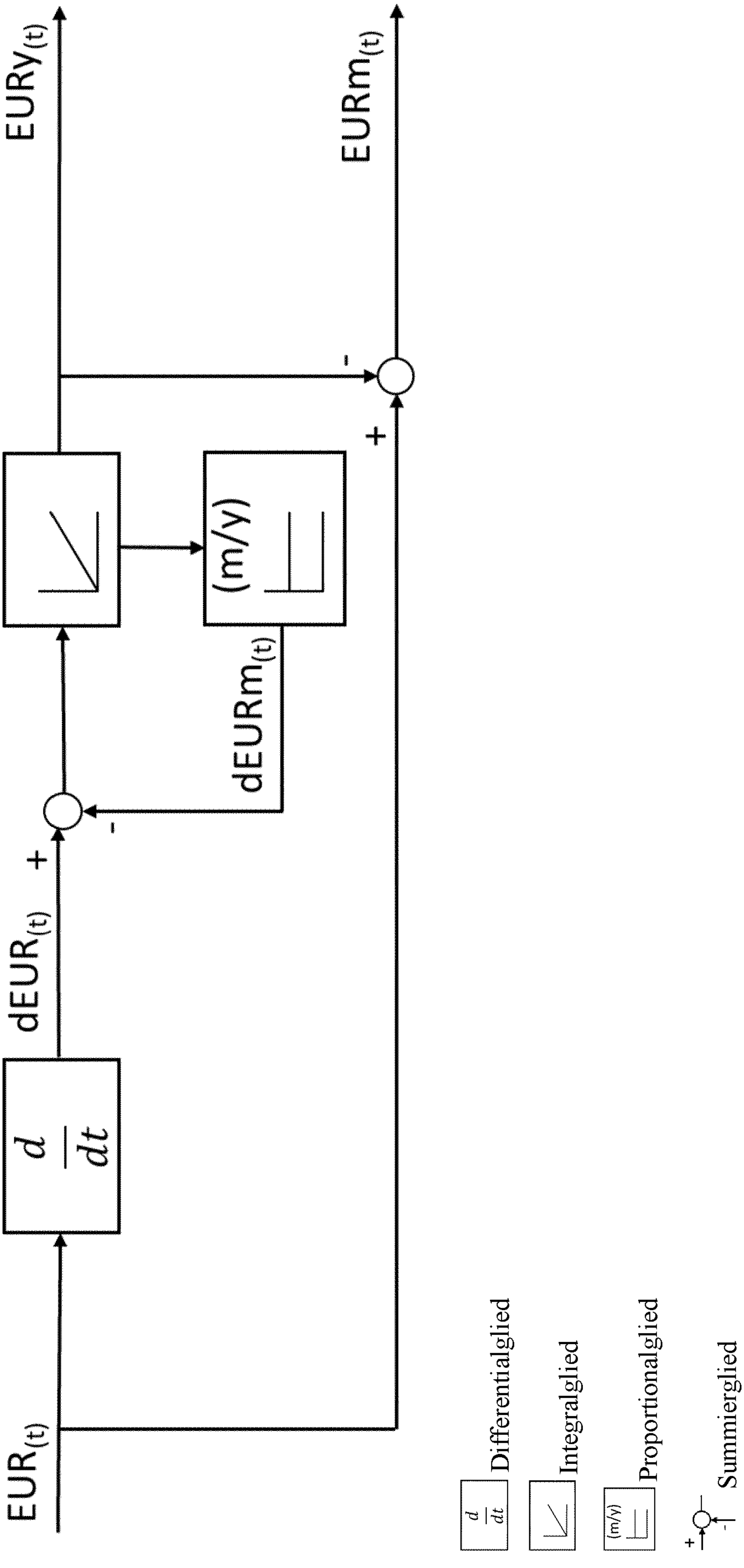
EURy(t)
EURm(t)
EUR(t)
dEUR(t)
dEURm(t)
(m/y)
Differentialglied
Integralglied
Proportionalglied
Summierglied

Figur 3

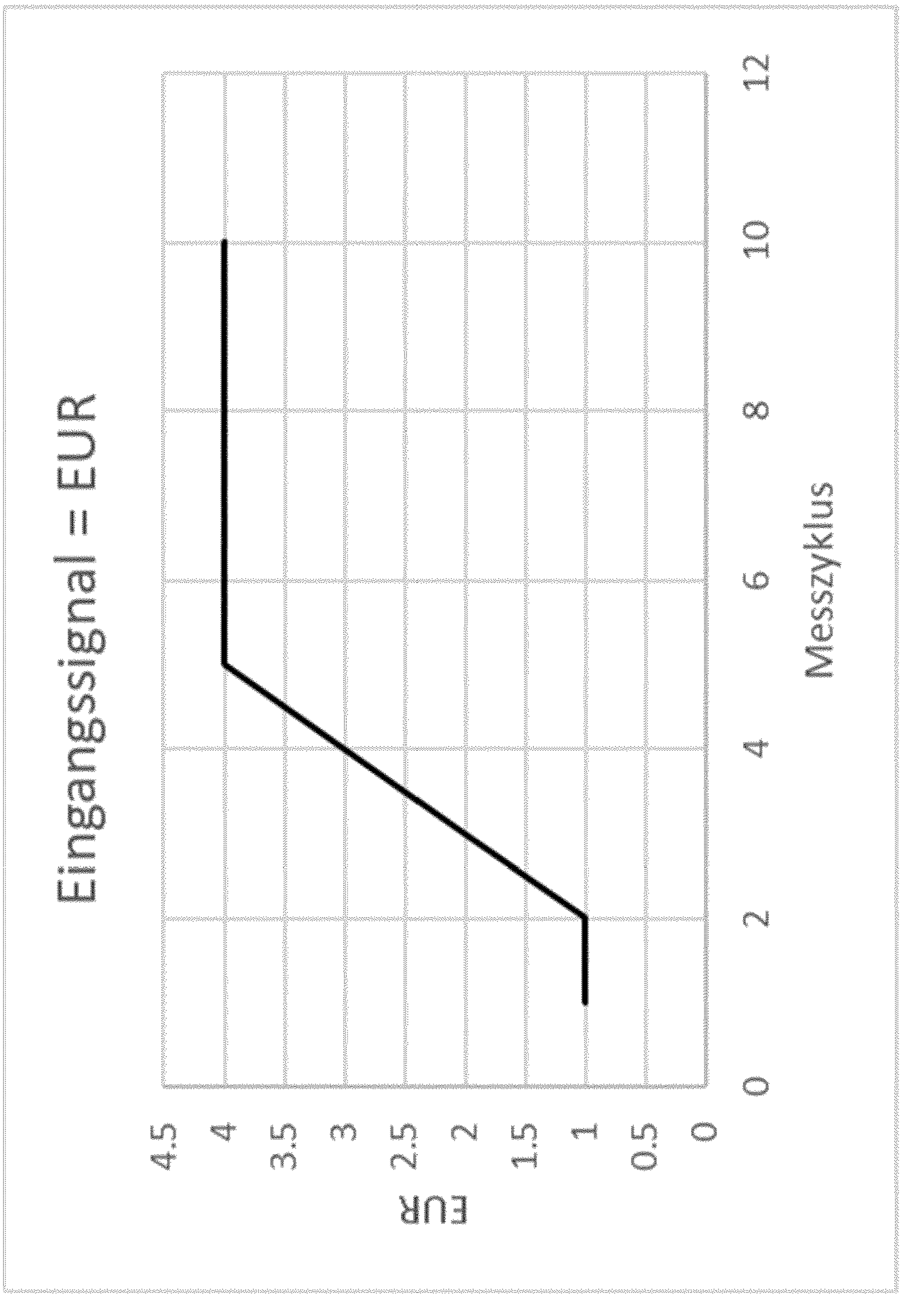
Eingangssignal = EUR
EUR
4.5
4
3.5
3
2.5
2
1.5
1
0.5
0
0
2
4
6
8
10
12
Messzyklus

Figur 4

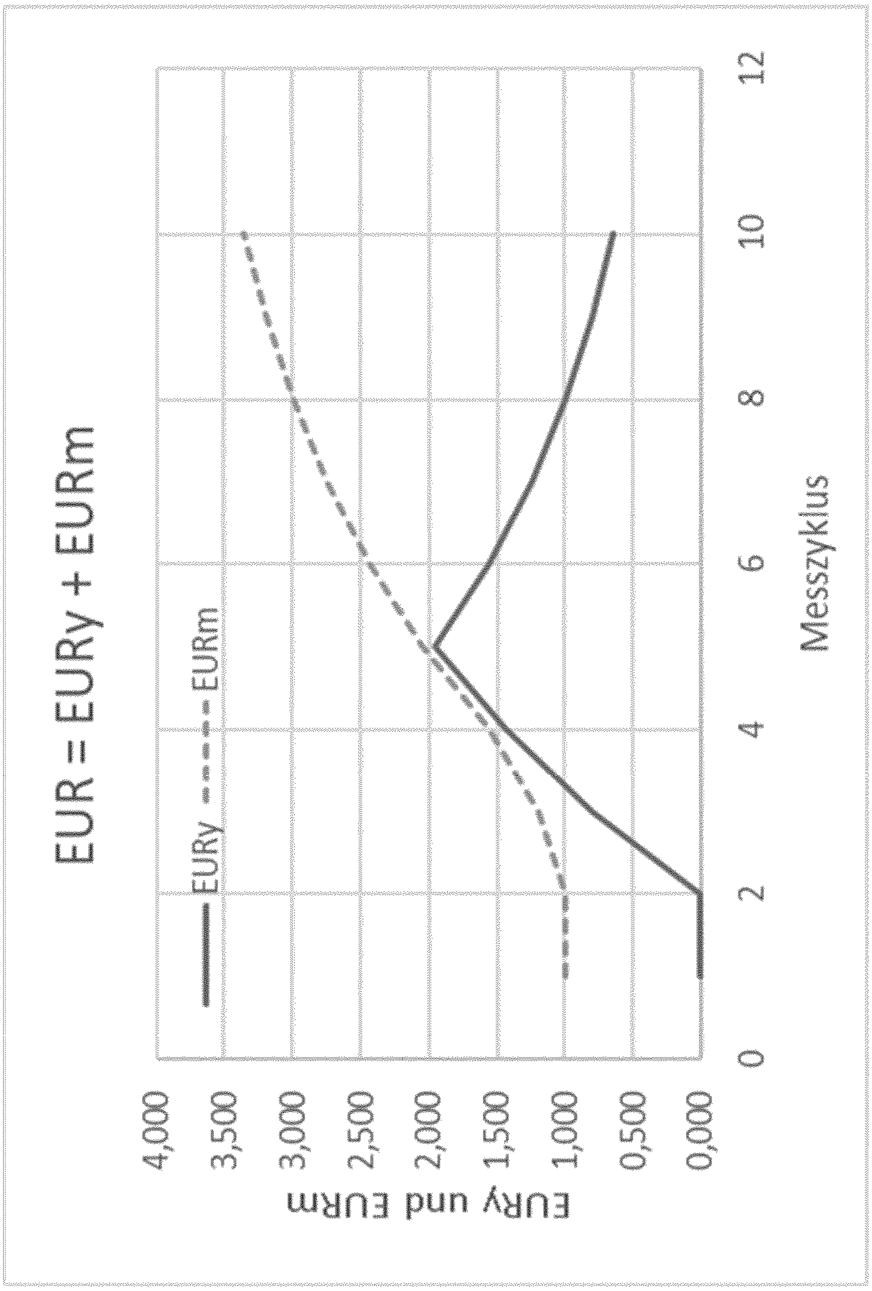
EUR = EURy + EURm
EURy
EURm
EURy und EURm
4,000
3,500
3,000
2,500
2,000
1,500
1,000
0,500
0,000
0
2
4
6
8
10
12
Messzyklus

Figur 5

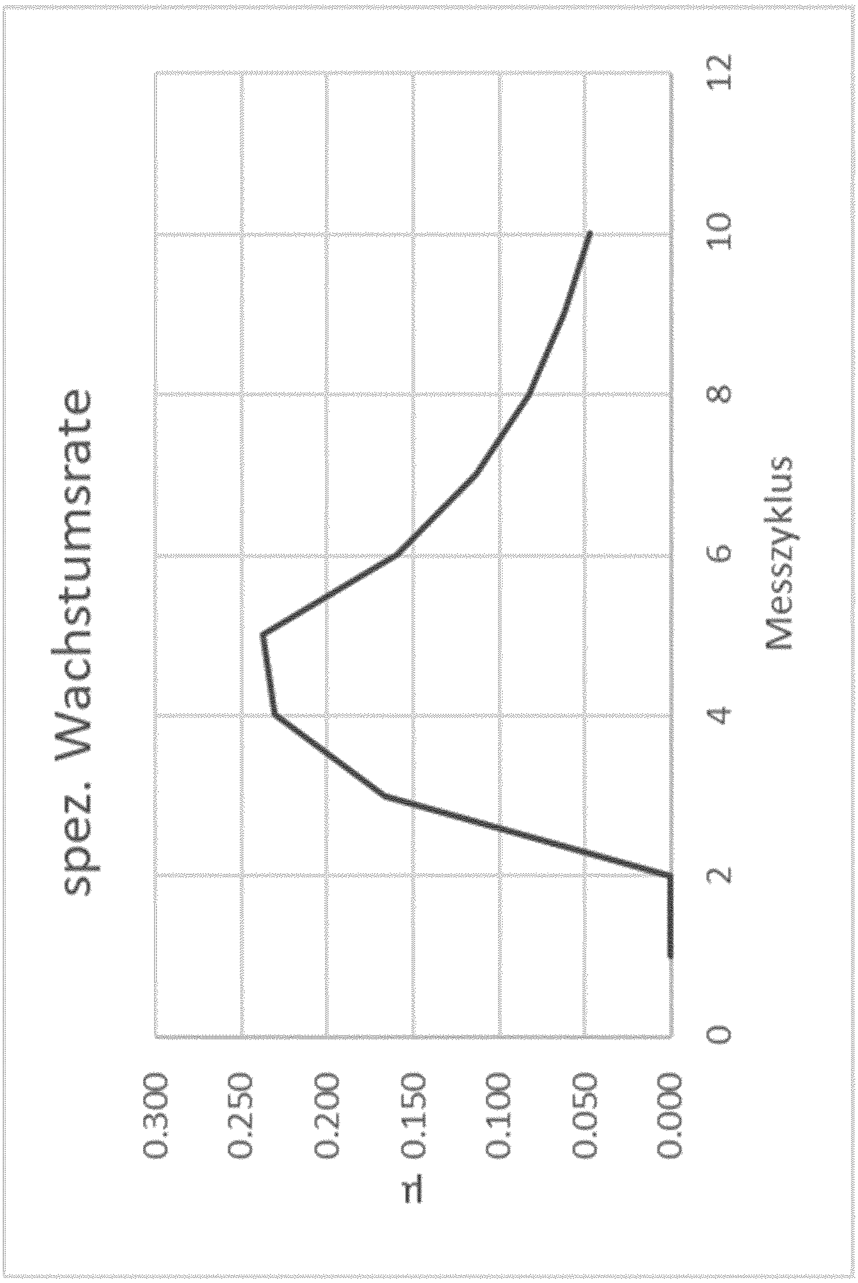
spez. Wachstumsrate
µ
0.300
0.250
0.200
0.150
0.100
0.050
0.000
0
2
4
6
8
10
12
Messzyklus

Figur 6

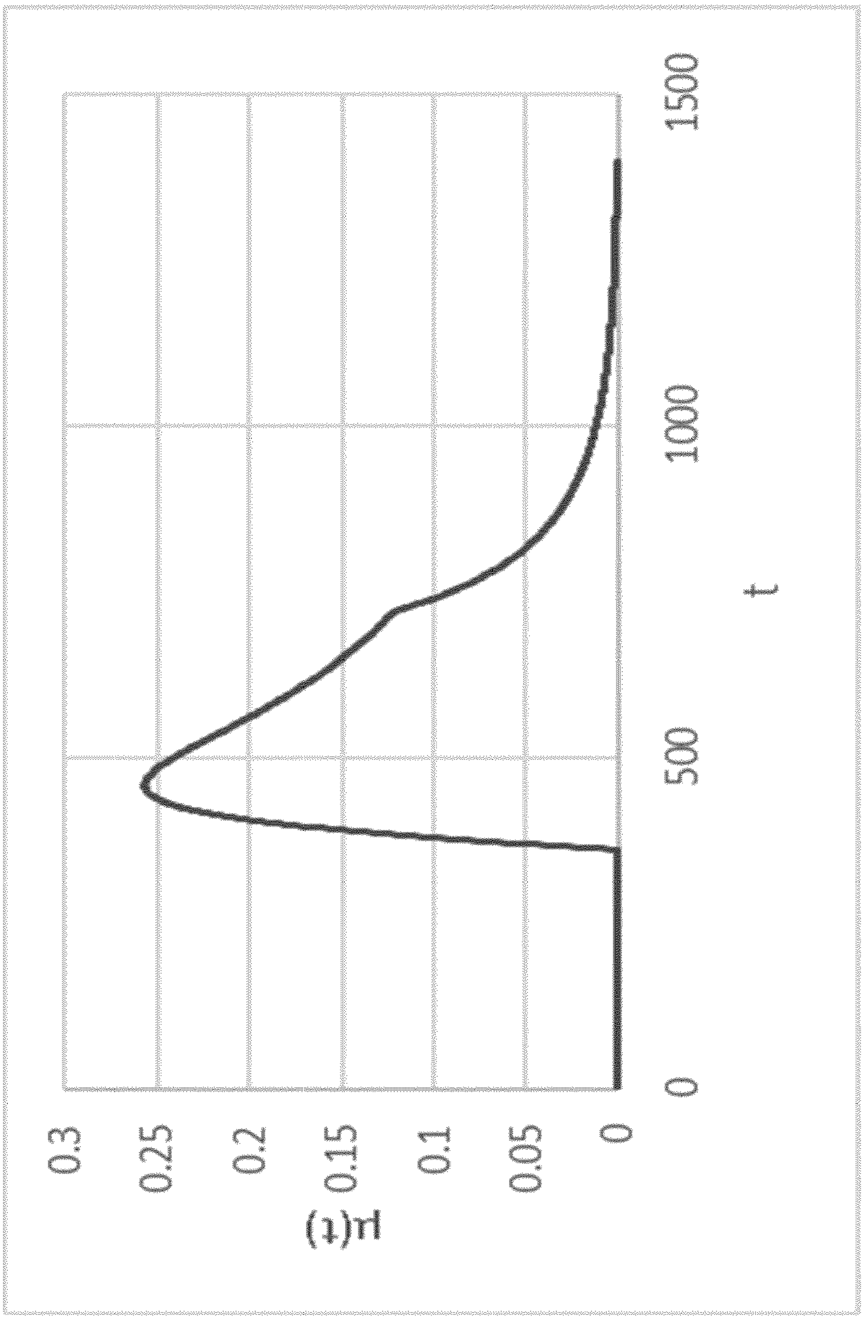

Figur 7

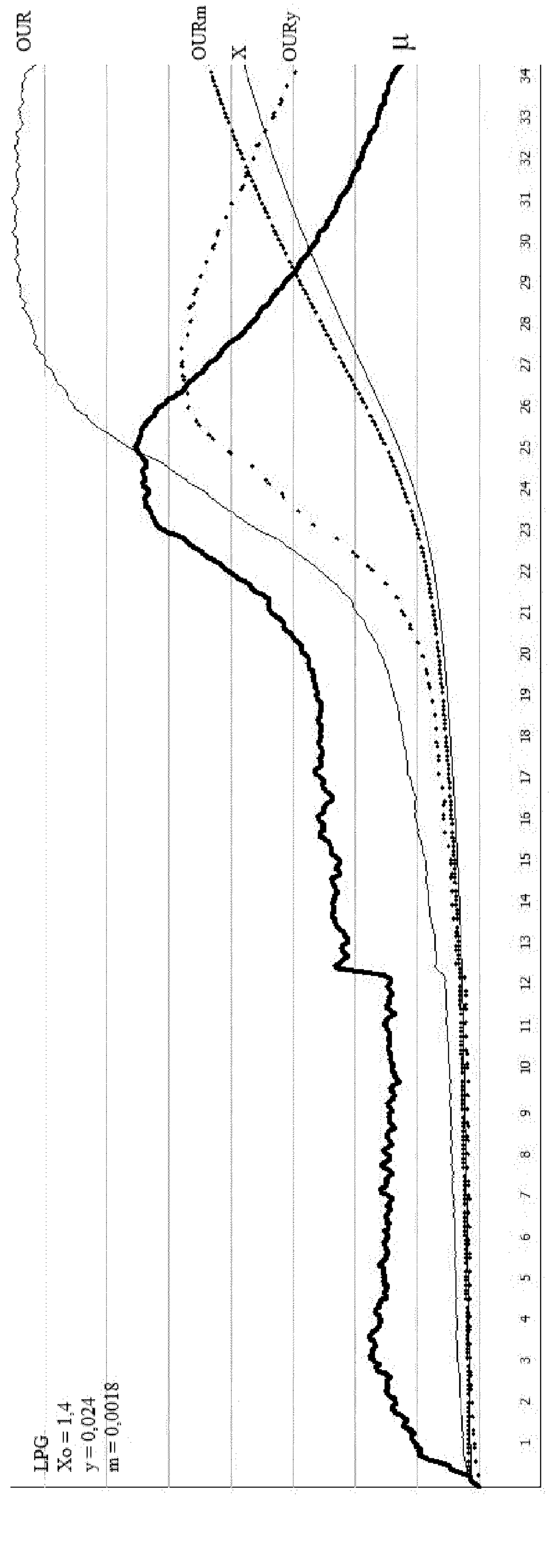
OUR
OURm
X
OURy
μ
LPG
Xo = 1,4
y = 0,024
m = 0,0018
1 2 3 4 5 6 7 8 9 10 11 12 13 14 15 16 17 18 19 20 21 22 23 24 25 26 27 28 29 30 31 32 33 34

Figur 8

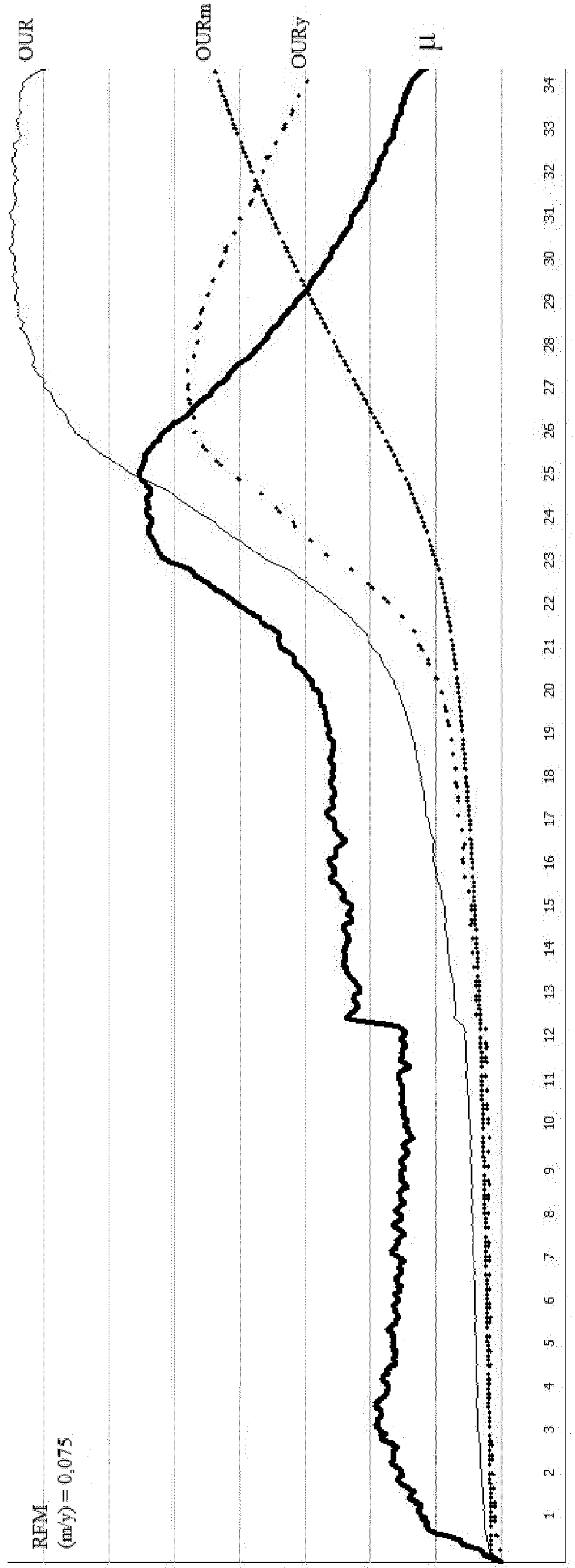
OUR
OURm
OURy
μ
RFM
(m/y) = 0,075
1 2 3 4 5 6 7 8 9 10 11 12 13 14 15 16 17 18 19 20 21 22 23 24 25 26 27 28 29 30 31 32 33 34

Europäisches Patentamt
European Patent Office
Office européen des brevets

# EUROPÄISCHER RECHERCHENBERICHT

Nummer der Anmeldung
EP 25 16 1326

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 2009/048816 A1 (SRINIVASA BABJI BUDDHI [IN] ET AL) 19. Februar 2009 (2009-02-19)<br>* Absätze [0002], [0006], [0011], [0012], [0015], [0017], [0029], [0057], [0063], [0065], [0070] - [0074], [0082]; Anspruch 1; Abbildungen 8-10 *<br>----- | 12<br>1-11,13, 14 | INV.<br>C12M1/34<br>C12M1/36<br>C12Q1/04 |
| Y | WO 2020/224779 A1 (INSILICO BIOTECHNOLOGY AG [DE]) 12. November 2020 (2020-11-12)<br>* Seite 1 *<br>* Seite 3, Absatz 4-6 *<br>* Seite 4, Absätze 1,3 *<br>* Seite 5, Absatz 3 *<br>* Seite 6, Absätze 1,2 *<br>* Seite 7, Absatz 1 *<br>* Seite 9, Absatz 2 *<br>* Seite 11, Absatz 4 *<br>* Seite 14, Absatz 3 *<br>----- | 1-11,13, 14 | |
| A | KRULL RAINER ET AL: "Analysis of reaction kinetics during chemostat cultivation ofSaccharomyces cerevisiaeusing a multiphase microreactor", BIOCHEMICAL ENGINEERING JOURNAL, ELSEVIER, AMSTERDAM, NL, Bd. 105, 8. Oktober 2015 (2015-10-08), Seiten 220-229, XP029290465, ISSN: 1369-703X, DOI: 10.1016/J.BEJ.2015.08.013<br>* Seite 4, Absatz 1 *<br>* Seite 5, Absatz 6 *<br>* Seite 6 *<br>* Seite 9, Absatz 6 *<br>* Seite 10, Absatz 5 *<br>* Seite 12, Absätze 7,8 *<br>* Seite 13, Absätze 1-3,7,8 *<br>-----<br>-/-- | 1-14 | RECHERCHIERTE SACHGEBIETE (IPC)<br>C12M<br>G01N<br>C12Q |

Der vorliegende Recherchenbericht wurde für alle Patentansprüche erstellt

1

| Recherchenort | Abschlußdatum der Recherche | Prüfer |
|---|---|---|
| Berlin | 31. Juli 2025 | Böhm, Ingo |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 25 16 1326

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | JUNKER B H ET AL: "Bioprocess monitoring and computer control: Key roots of the current PAT initiative", BIOTECHNOLOGY AND BIOENGINEERING, JOHN WILEY, HOBOKEN, USA, Bd. 95, Nr. 2, 24. August 2006 (2006-08-24), Seiten 226-261, XP071052463, ISSN: 0006-3592, DOI: 10.1002/BIT.21087 * Seite 9 * ----- | 1-14 | |
| A | EP 3 296 387 B1 (SIEMENS AG [DE]) 15. Mai 2019 (2019-05-15) * Absätze [0001], [0003], [0007] - [0009], [0023], [0024]; Ansprüche * ----- | 1 | |

RECHERCHIERTE SACHGEBIETE (IPC)

Der vorliegende Recherchenbericht wurde für alle Patentansprüche erstellt

1

| Recherchenort | Abschlußdatum der Recherche | Prüfer |
|---|---|---|
| Berlin | 31. Juli 2025 | Böhm, Ingo |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 2 von 2

## ANHANG ZUM EUROPÄISCHEN RECHERCHENBERICHT ÜBER DIE EUROPÄISCHE PATENTANMELDUNG NR.

EP 25 16 1326

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-07-2025

| Im Recherchenbericht angeführtes Patentdokument | Datum der Veröffentlichung | Mitglied(er) der Patentfamilie | Datum der Veröffentlichung |
|---|---|---|---|
| US 2009048816 A1 | 19-02-2009 | CN 101370926 A | 18-02-2009 |
| | | EP 2001991 A1 | 17-12-2008 |
| | | US 2009048816 A1 | 19-02-2009 |
| | | WO 2007085880 A1 | 02-08-2007 |
| WO 2020224779 A1 | 12-11-2020 | CN 114502715 A | 13-05-2022 |
| | | EP 3966310 A1 | 16-03-2022 |
| | | JP 7554774 B2 | 20-09-2024 |
| | | JP 2022537799 A | 30-08-2022 |
| | | SG 11202112113P A | 29-11-2021 |
| | | US 2022213429 A1 | 07-07-2022 |
| | | WO 2020224779 A1 | 12-11-2020 |
| EP 3296387 B1 | 15-05-2019 | CN 107832582 A | 23-03-2018 |
| | | EP 3296387 A1 | 21-03-2018 |
| | | US 2018082011 A1 | 22-03-2018 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- *Practical Solutions for Specific Growth Rate Control Systems in Industrial Bioreactors*, 2019
- *An oxygen-uptake-rate-based estimator of the specific growth rate in Escherichia coli BL21 strains cultivation process*, 2021
- **M. M. SILVEIRA** ; **M. A. B. MOLINA**. Indirect estimation of Bacillus thuringiensis var. israelensis biomass concentration using oxygen balance data. *Brazilian Journal of Chemical Engineering*, 2005, vol. 22 (4), 495-500
- **D. W. ZABRISKIE** ; **A. E. HUMPHREY**. Real-time estimation of aerobic batch fermentation biomass concentration by component balancing. *AIChE Journal*, 1978, vol. 24 (1), 138-146
- **R. LUEDEKING** ; **E. L. PIRET**. A kinetic study of the lactic acid fermentation. Batch process at controlled pH. *Journal of Biochemical and Microbiological Technology and Engineering*, 1959, vol. 1 (4), 393-412
- **KRAUSCH et al.** Model-Based Characterization of E. coli Strains with Impaired Glucose Uptake. *Bioengineering*, 2023, vol. 10, 808, https: //doi.org/10.3390/-bioengineering10070808